(19) Europäisches Patentamt
European Patent Office
Office européen des brevets

(11) Numéro de publication: **0 126 669 B1**

(12) **FASCICULE DE BREVET EUROPEEN**

(45) Date de publication du fascicule du brevet: **27.08.86**

(51) Int. Cl.⁴: **C 07 C 17/12**, B 01 J 31/02

(21) Numéro de dépôt: **84400860.7**

(22) Date de dépôt: **27.04.84**

(54) **Dérivé N substitué de la phénothiazine comme catalyseur de chloration sur le noyau des hydrocarbures aromatiques.**

(30) Priorité: **29.04.83 FR 8307117**

(43) Date de publication de la demande: **28.11.84 Bulletin 84/48**

(45) Mention de la délivrance du brevet: **27.08.86 Bulletin 86/35**

(84) Etats contractants désignés: **BE CH DE FR GB IT LI NL**

(56) Documents cités:
**FR - A - 2 319 607**
**GB - A - 873 066**
**GB - A - 1 312 950**
**GB - A - 2 054 571**
**US - A - 4 024 198**
**US - A - 4 243 782**

(73) Titulaire: **ATOCHEM, 4 & 8, Cours Michelet La Défense 10, F-92800 Puteaux (FR)**

(72) Inventeur: **Commandeur, Raymond, Le Rocher Avenue de Venaria, F-38220 Vizille (FR)**
Inventeur: **Gurtner, Bernard, 5, Boulevard Agutte Sembat, F-38000 Grenoble (FR)**
Inventeur: **Mathais, Henri Hameau de St Didier, Villa No. 2 Chemin de l'Indiennerie, F-69370 Saint-Didier-au-Mont-d'Or (FR)**

(74) Mandataire: **Foiret, Claude et al, ATOCHEM Département Propriété Industrielle, F-92091 Paris la Défense 10 Cédex 42 (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).



ACTORUM AG

## Description

La présente invention concerne un système catalytique constitué d'acides de Lewis associés aux dérivés N substitués de la phénothiazine pour la chloration sur le noyau des hydrocarbures aromatiques.

Lors de la fabrication industrielle des hydrocarbures aromatiques chlorés par chloration catalytique par le chlore de ces hydrocarbures, il se forme des isomères para mais également ortho dont l'intérêt économique n'est généralement que très limité. Pour favoriser l'orientation en para, on utilise habituellement un système catalytique constitué d'une combinaison d'un acide de Lewis avec le soufre ou les chlorures de soufre ou encore avec le thianthrène, comme dans le brevet des Etats-Unis d'Amérique N° 4024198 ou la phénoxthine comme dans le brevet japonais N° 81 110630. Le thianthrène et la phénoxthine présentent l'inconvénient d'être de commercialisation peu courante et de ne s'appliquer qu'à la chloration des alkylbenzènes et plus particulièrement du toluène.

Selon l'invention, la formation des isomères en para se trouve améliorée lors de la chloration des hydrocarbures aromatiques quand on associe au catalyseur à base d'acide de Lewis un dérivé N substitué de la phénothiazine de formule

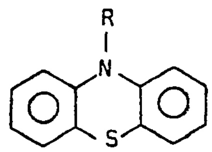

dans laquelle −R représente:

soit 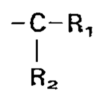

avec

$-R_1$ représentant: =O, =S, 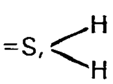, 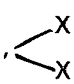

X étant l'halogène Br ou Cl

et $-R_2$ représentant un radical aryle, l'halogène Br ou Cl ou le radical $-CH_xX_y$ dans lequel X est l'halogène Br ou Cl avec $x = 0$ à 2, $y = 1$ à 3 et $x + y = 3$,

soit un radical aryle.

Parmi les radicaux aryles sont particulièrement recommandés le phényle, le benzyle, le biphényle, le tolyle. Pour −R, le radical chlorocarbonyle est préféré.

L'efficacité du système catalytique provenant de façon inévitable du radical −R substitué, il va de soi qu'on entend également sous la dénomination phénothiazine les phénothiazines possédant au moins une quelconque substitution et plus particulièrement du chlore sur au moins un des cycles aromatiques, une telle substitution ne pouvant avoir qu'un rôle secondaire sur l'effet catalytique.

Ce dérivé N substitué de la phonothiazine est particulièrement intéressant dans la mesure où il est fabriqué facilement à partir d'un produit industriel peu coûteux: la phénothiazine. Il peut être obtenu directement:

- par acylation par les chlorures d'acide, comme décrit dans: J. Chem. Soc. 1954, 2577-9; CA *49* 8987,
- par transalkylation avec les oxalates, comme décrit dans Bull. Soc. Chim. France 1960, 112-24; CA *55* 2651,
- par benzylation par les chlorures de benzyle, comme décrit dans le brevet britannique 873066,
- par arylation par les dérivés bromés aromatiques, comme décrit dans J. Org. Chem. 23, 628-9 (1958); CA *52* 17277,
- par réaction du phosgène avec la phénothiazine en vue d'obtenir la N chlorocarbonylphénothiazine, comme décrit dans le brevet français 1192168.

Ces produits, faciles à synthétiser, sont particulièrement intéressants dans la mesure où ils conduisent à une très bonne sélectivité, entre autres en paradichlorobenzène dans la chloration du benzène. Leur activité catalytique est excellente et les réactions ne nécessitent qu'une faible quantité de catalyseur. D'autre part, leur bonne stabilité permet de séparer les composés de la chloration par distillation et de recycler le résidu contenant le catalyseur, cette opération étant renouvelable plusieurs fois.

Le dérivé N substitué de la phénothiazine associé comme catalyseur aux acides de Lewis permet de réduire la substitution en ortho dans la chloration par le chlore des hydrocarbures aromatiques. Les acides de Lewis utilisés dans ce type de réaction sont connus. Parmi les plus courants, on peut citer les oxydes, oxychlorures, sulfates et, plus particulièrement, les chlorures métalliques, les principaux métaux étant le Ti, Zr, Hf, Nb, Ta, Mo, W, Ga, Sn et surtout Fe et Sb. Dans la réaction de chloration, la teneur en acide de Lewis dans l'hydrocarbure aromatique peut varier de 0,01% à 3% en poids et plus usuellement de 0,02 à 0,08%, le rapport molaire entre l'acide de Lewis et le dérivé N substitué de la phénothiazine pouvant varier de 0,1/1 à 10/1 et plus usuellement de 0,3/1 à 2/1.

La réaction de chloration s'effectue de façon classique par introduction de chlore dans un réacteur contenant l'hydrocarbure aromatique et le système catalytique, la température étant comprise entre −20 et 100° C et habituellement entre 20 et 60° C.

Le système catalytique convient à la chloration de tous les hydrocarbures aromatiques. Il est cependant particulièrement recommandé, pour la chloration du benzène, des hydrocarbures aromatiques possédant un substituant ortho et para orienteur à caractère électronégatif comme −Cl, −F, −Br, des hydrocarbures aromatiques possédant un sustituant ortho et para orienteur à caractère électropositif comme $-CH_3$; $C_2H_5$, $nC_3H_7$, iso $C_3H_7$, n-butyle, terbutyle ou alcoxy, des hydrocarbures aromatiques possédant un substituant méta orienteur comme $-NO_2$, 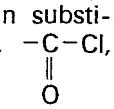

$-CCl_3$, $-CF_3$. Il est également recommandé pour la chloration des hydrocarbures aromatiques possédant plusieurs substituants ortho et para orienteurs comme par exemple l'orthoxylène ou l'orthochlorotoluène.

Les exemples suivants sont donnés à titre illustratif et non limitatif. La réaction est effectuée dans un réacteur en verre muni d'un agitateur, d'un dispositif d'introduction de chlore gazeux, d'un système réfrigérant et d'une sortie des gaz de réaction avec absorbeur. On dissout le système catalytique dans l'hydrocarbure à traiter. Après le temps de réaction nécessaire pendant lequel la température est maintenue constante, on dégaze le milieu réactionnel au moyen d'un gaz inerte. Les produits obtenus sont analysés par chromatographie en phase vapeur. La sélectivité du système catalytique est déterminée par le rapport r de la concentration de l'isomère para sur la concentration de l'isomère ortho pour les substituants ortho et para orienteurs.

*Exemple 1:*

Dans une molécule-gramme de toluène, on place 10 mg de chlorure ferrique anhydre et 26 mg de N chlorocarbonylphénothiazine. On introduit le chlore à raison de 0,166 mole/h tout en maintenant la température à 30° C. Au bout de 5 heures de chloration, l'analyse du produit de réaction indique la composition suivante en poids: toluène non transformé = 13,16%; o-chlorotoluène = 39,77; parachlorotoluène = 47,15%; dichlorotoluène = 0,31%. Le rapport r est de 1,20.

Dans la chloration catalysée par $FeCl_3-S_2Cl_2$, ce rapport n'est que de 0,9 seulement.

*Exemple 2:*

Dans une molécule-gramme de toluène contenant 0,5% de $SbCl_3$ et 1,48% de N-chlorocarbonylphénothiazine, on introduit du chlore gazeux à raison de 0,25 mole/h tout en maintenant la température entre 40 et 50° C. Au bout de 2 heures de chloration, l'analyse du produit de réaction indique un rapport r = 1,13.

*Exemple 3:*

En opérant sur 3 moles de benzène contenant 250 ppm de $FeCl_3$ et 513 ppm de N-chlorocarbonylphénothiazine, on introduit du chlore gazeux à la vitesse de 1 mole/h. La température est maintenue à 60° C. Au bout de 4,5 heures, l'analyse du produit de réaction indique les valeurs suivantes:

| | |
|---|---|
| – Benzène | 1,43% |
| – Monochlorobenzène | 54,98% |
| – Dichlorobenzène ortho | 7,52% |
| – Dichlorobenzène méta | 0,13% |
| – Dichlorobenzène para | 35,88% |
| – Trichlorobenzènes | 0,05% |

avec un rapport r = 4,77.

Dans la chloration catalysée par $FeCl_3-S_2Cl_2$, ce rapport n'est que de 3.

*Exemple 4:*

En opérant comme dans l'exemple 3, mais en présence de 192 ppm de $SbCl_3$ et 337 ppm de N-chlorocarbonylphénothiazine, le rapport r = 4,80.

*Exemple 5:*

Dans 6 moles de chlorobenzène contenant 0,22% de $FeCl_3$ et 0,57% de N-chlorocarbonylphénothiazine, on introduit du chlore gazeux au débit de 1 mole/h en maintenant la température à 20° C. Au bout de 5 heures de réaction, l'analyse du milieu réactionnel est la suivante:

| | |
|---|---|
| – Monochlorobenzène | 32,06% |
| – Dichlorobenzène ortho | 8,64% |
| – Dichlorobenzène méta | 0,08% |
| – Dichlorobenzène para | 59,22% |
| – Trichlorobenzènes | 0 % |

avec un rapport r = 6,85.

*Exemple 6:*

Dans une mole d'orthochlorotoluène contenant 110 mg de $FeCl_3$ et 182 mg de N-chlorocarbonylphénothiazine, on introduit 0,166 mole/h de chlore gazeux durant 3 heures en maintenant la température à 60° C. L'analyse du milieu réactionnel est la suivante:

| | |
|---|---|
| – Orthochlorotoluène | 45,9 % |
| – Dichlorotoluène 2-5 | 35,38% |
| 2-6 | 4,14% |
| 2-4 | 8,79% |
| 2-3 | 4,22% |
| – Trichlorotoluènes | 1,37% |

La teneur en isomère 2-5 par rapport à l'ensemble des isomères dichlorés est de 67,5%, elle n'est que de 60% en utilisant S ou $S_2Cl_2$ associé à $FeCl_3$.

*Exemple 7:*

Dans une mole de paraxylène contenant 0,5% de $SbCl_3$ et 1,48% de N-chlorocarbonylphénothiazine, on introduit 1 mole/h de chlore durant 1,5 heure à 40° C. On obtient un mélange d'isomères contenant 55,31% de 2 chloro, 32,26% de 2-5 dichloro et 6,56% de 2-3 dichloro. Le rapport isomérique 2-5/2-3 est de 4,91, il n'est que de 2,48 avec $SbCl_3$ et de 4,19 avec la combinaison $SbCl_3-S$.

*Exemple 8:*

En opérant avec 1 mole d'orthoxylène contenant les mêmes quantités de catalyseurs que dans l'exemple 7, mais en introduisant 0,25 mole/h de chlore durant 2 heures, à 35° C, on obtient un mélange réactionnel contenant 16,18% d'isomère chloré en position 3 et 37,03% d'isomère en position 4, d'où un rapport isomérique r = 2,3. Ce rapport n'est que de 1,65 avec $SbCl_3$ seul et de 1,95 avec la combinaison $SbCl_3-S$.

*Exemple 9:*

On chlore du toluène dans les conditions de l'exemple 2 en présence de 0,5% de $SbCl_3$ et d'une

quantité de dérivé N substitué de la phénothiazine calculée pour avoir un rapport moléculaire soufre-antimoine constant. Le tableau ci-après résume les résultats obtenus en fonction du dérivé N substitué utilisé. L'abréviation PHT a été utilisée pour symboliser la molécule de phénothiazine.

| *Dérivé de la phénothiazine* | | *r = para/ortho* |
|---|---|---|
| (1) | 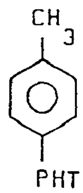 | 1,17 |
| (2) | 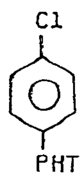 | 1,0 |
| (3) | 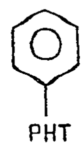 | 1,16 |
| (4) | 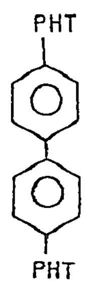 | 1,26 |
| (5) | 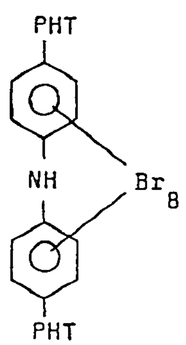 | 1,07 |
| (6) | 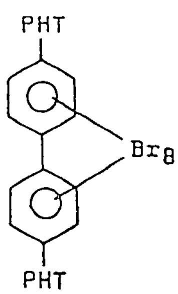 | 1,02 |

| *Dérivé de la phénothiazine* | | *r = para/ortho* |
|---|---|---|
| (7) | 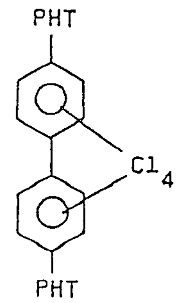 | 1,09 |
| (8) | 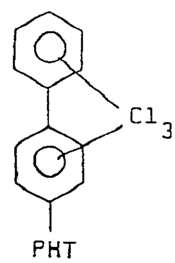 | 1,17 |
| (9) | 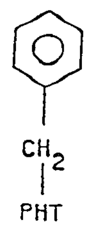 | 1,04 |
| (10) | 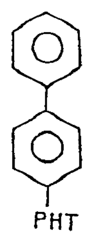 | 1,26 |
| (11) | 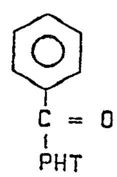 | 1,0 |

*Exemple 10:*

On chlore du benzène, en opérant selon l'exemple 3 mais en remplaçant le N chlorocarbonylphénothiazine par 667 ppm de 2 chloro N chlorocarbonylphénothiazine:

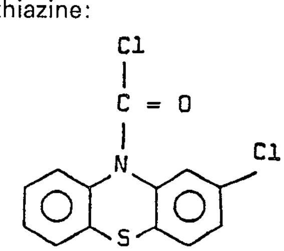

Le rapport r obtenu sur le produit final est égal à 4,6.

*Exemple 11:*

Dans les conditions de l'exemple 2, on chlore du toluène en présence de 0,5% de chlorure de niobium et de 1,7% du dérivé 10 de l'exemple 9.

Le rapport r obtenu sur le produit final est égal à 1,21.

*Exemple 12:*

Dans les conditions de l'exemple 2, on chlore du toluène en présence de 0,4% de chlorure de gallium et de 1,76% du dérivé 4 de l'exemple 9.

Le rapport r obtenu sur le produit final est égal à 1,23.

*Exemple 13:*

On introduit le chlore avec un débit de 1 mole/h pendant 4 h 30 à la température de 60°C dans 3 moles de benzène contenant 556 ppm de $FeCl_3$ et 1346 ppm de N chlorocarbonylphénothiazine. Après fin de réaction, le produit brut est soumis à une distillation dans le réacteur après équipement d'une colonne de 5 plateaux. Le chauffage est assuré par un bain d'huile à 120°C et on utilise un vide 16 mm de mercure. Après fin de distillation, on charge 3 moles de benzène dans le réacteur contenant le résidu de la distillation. On effectue ainsi 4 recyclages. Les résultats obtenus sont inscrits dans le tableau suivant:

| N° essai | HCl dégagé (mole) | $-Cl_2$ non réagi | p/o |
|---|---|---|---|
| 1 | 4,43 | 0,024 | 5,04 |
| 2 | 4,5 | 0,32 | 4,59 |
| 3* | 4,38 | 0,054 | 4,88 |
| 4** | 4,37 | 0,07 | 4,82 |
| 5*** | 4,44 | 0,06 | 4,82 |

* + 111 ppm $FeCl_3$
** + 56 ppm $FeCl_3$
*** + 56 ppm $FeCl_3$

*Exemple 14:*

Dans 6 moles de nitrobenzène contenant 1% de $SbCl_3$ et 1,15% de chlorocarbonylphénothiazine, on introduit le chlore pendant 4 h avec un débit de 1 mole/h à la température de 60°C. On ajoute ensuite 1% de $SbCl_3$ et on poursuit l'introduction du chlore pendant encore 3 h 30 de manière à obtenir la réaction de 50% du nitrobenzène. L'analyse CPG des monochloronitrobenzènes conduit à un rapport isomérique méta/para = 32,2 et méta/ortho = 14,2, alors que ces rapports sont respectivement de 21,2 et 7,14 dans la chloration classique catalysée par $FeCl_3$ + iode.

*Exemple 15:*

Dans 1,5 mole de dichlorobenzène-1,2, contenant 0,2% de $FeCl_3$ et 0,41% de chlorocarbonylphénothiazine, on introduit le chlore pendant 1 h 30 avec un débit de 0,5 mole/h à la température de 60°C. L'analyse du produit de réaction conduit à un rapport des isomères $\frac{1\text{-}2\text{-}4}{1\text{-}2\text{-}3} = 7{,}1$ alors que, dans la réaction catalysée par $FeCl_3-S_2Cl_2$, ce rapport est égal à 3,7.

## Revendications

1. Système catalytique à base d'acide de Lewis pour la chloration sur le noyau des hydrocarbures aromatiques, caractérisé en ce qu'il contient un dérivé N substitué de la phénothiazine de formule

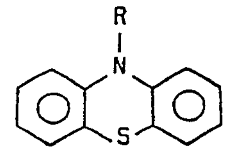

dans laquelle −R représente:

soit 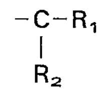

avec

−R₁ représentant: =O, =S, 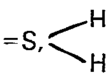, 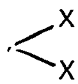

X étant l'halogène Br ou Cl

et −$R_2$ représentant un radical aryle, l'halogène Br ou Cl ou le radical $-CH_xX_y$ dans lequel X est l'halogène Br ou Cl avec x = 0 à 2, y = 1 à 3 et x + y = 3,

soit un radical aryle.

2. Système catalytique selon la revendication 1, caractérisé en ce que le dérivé N substitué de la phénothiazine contient au moins une quelconque substitution sur au moins un des cycles aromatiques.

3. Système catalytique selon la revendication 1, caractérisé en ce que le rapport molaire entre l'acide de Lewis et le dérivé N substitué de la phénothiazine est compris entre 0,1/1 et 10/1.

## Patentansprüche

1. Katalytisches System auf Basis einer Lewis-Säure zur Kernchlorierung von aromatischen Kohlenwasserstoffen, dadurch gekennzeichnet, dass es ein N-substituiertes Phenothiazinderivat der Formel

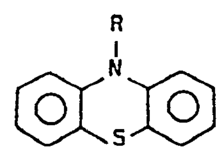

enthält, in der R ein Arylreste oder 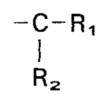

ist, wobei $R_1$ =O, =S, 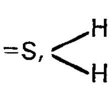 oder 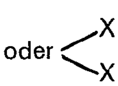

bedeutet, X Br oder Cl ist,

$R_2$ ein Arylrest, Br oder Cl oder der Rest $-CH_xX_y$ ist, in dem X Br oder Cl, x einen Wert von 0 bis 2, y einen Wert von 1 bis 3 bedeuten, und x + y = 3 ist.

2. Katalytisches System nach Anspruch 1, dadurch gekennzeichnet, dass das N-substituierte

Phenothiazinderivat mindestens einen Substituenten an mindestens einem der aromatischen Ringe enthält.

3. Katalytisches System nach Anspruch 1, dadurch gekennzeichnet, dass das Molverhältnis zwischen der Lewis-Säure und dem N-substituierten Phenothiazinderivat zwischen 0,1/1 und 10/1 liegt.

## Claims

1. Catalyst system based on a Lewis acid for the ring chlorination of aromatic hydrocarbons, characterized in that it contains an N-substituted phenothiazine derivative of formula:

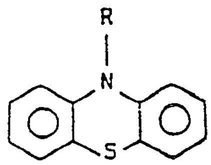

in which R denotes either 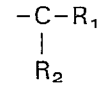

with $R_1$ denoting:

=O, =S, 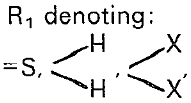 X being the halogen Br or Cl, and $R_2$ denoting an aryl radical, the halogen Br or Cl, or the radical $-CH_xX_y$ in which X is the halogen Br or Cl with x = 0 to 2, y = 1 to 3 and x + y = 3

or an aryl radical.

2. Catalyst system according to Claim 1, characterized in that the N-substituted phenothiazine derivative contains at least one substituent of any kind on at least one of the aromatic rings.

3. Catalyst system according to Claim 1, characterized in that the molar ratio of the Lewis acid to the N-substituted phenothiazine derivative is between 0.1/1 and 10/1.